# EUROPEAN PATENT APPLICATION

(11) **EP 4 817 559 A1**
(43) Date of publication of application: **07.10.2026**
(21) Application number: 26169363.4
(22) Date of filing: 31.03.2026
(51) Int. Cl.: A61F 2/14, A61F 2/00

(54) **COLORED OPHTHALMOLOGICAL IMPLANT AND METHOD OF MANUFACTURING A COLORED OPHTHALMOLOGICAL IMPLANT**

(30) Priority: 03.04.2025 CH 3352025
(71) Applicant: Ziemer Ophthalmic Systems AG, 2562 Port (CH)
(72) Inventor: RATHJEN, Christian, 28197 Bremen (DE); STEINLECHNER, Michael, 8006 Zürich (CH)
(74) Representative: Rentsch Partner AG

(57) **Abstract**

The present disclosure relates to an colored ophthalmological implant (1) for arranging within a patient's eye and to a method of manufacturing the colored ophthalmological implant (1), the colored ophthalmological implant (1) comprising a base layer (10), which is made of ophthalmological tissue (12) and eye colorant (100), which is configured to color the ophthalmological implant (1), wherein the eye colorant (100) is arranged to color the base layer (10), thereby generating the desired colored ophthalmological implant (1).

## Description

### FIELD OF THE DISCLOSURE

The present disclosure relates to a colored ophthalmological implant for positioning or arranging in a patient's eye, to a method of manufacturing the colored ophthalmological implant and to an ophthalmological system for surgical treatment of a patient's eye. In particular the present disclosure relates to a colored ophthalmological implant for positioning or arranging in a patient's eye comprising a base layer and eye colorant, to a method of manufacturing the colored ophthalmological implant comprising the steps of providing the tissue, processing the base layer out of the provided tissue and coloring the processed base layer and to an ophthalmological system for surgical treatment of a patient's eye comprising an ophthalmological device and an implantation device.

### BACKGROUND OF THE DISCLOSURE

Each person has an individual iris color distribution and iris color structure. The individual color and structure of the iris is determined by an individual composition of pigments across the iris of the eye.

Different diseases, defects or conditions of the iris and the eye may affect or change the color of the iris or may affect the iris color distribution or the appearance of the iris. Further, it is possible that congenital diseases or defects affect the properties and geometrical extensions of the iris of the eye, such that the iris of the patient deviates from a normal ring shape. Such congenital diseases are for example albinism, coloboma or aniridia, further congenital diseases are also conceivable.

Further, other diseases or conditions may affect the capabilities of the human eye, in particular the quality of vision. To improve the quality of vision, it can be advantageous to modify the depth of field. A typical condition of the human eye associated with aging is the progressively worsening ability to focus clearly on close objects. A physiological insufficiency of optical accommodation typically manifests in difficulty in reading small print, which results in having to hold reading material farther away. An increase in depth of field of the optical vision can therefore help to counteract the insufficient optical accommodation properties of the eye.

A known treatment for problems with the depth of field is refractive surgery to create multifocal corneas. Results are, however, more variable and some people have a decrease in visual acuity. Another treatment option is corrective lenses, which provide correction over a range as high as +4.0 diopters. Another treatment option is to use a pinhole diaphragm, which is placed in front of the patient's eye to increase the depth of field. Common pinhole diaphragms are e.g. lenses or glasses.

The visual appearance of the iris may be amended by placing in the cornea of the respective eye, thus in front of the iris with respect to the visual axis of the eye, ink, such that the ink covers partially or entirely the natural color of the iris. Such a method is conventionally performed via a controlled chirurgical intervention. An ophthalmological laser device, for example a femtosecond laser, cuts a circular intrastromal pocket in the cornea of the eye of the patient and the desired ink is inserted into the pocket of the cornea. With such a chirurgical operation, it is possible to change partially or entirely the visual appearance of the iris color of the patient.

This ink, which has the desired color, is inserted in the intrastromal pocket. The ink stays in its liquid form in the intrastromal pocket in the cornea of the eye of the patient. The conventionally used ink may bleach in the sun or may change or loses its brightness and color / visual appearance over time. Further, it is conceivable that the ink penetrates over time into additional areas of the cornea of the eye, which may lead to an undesired visual appearance of the treated eye. Further, there may be adverse reactions of the ink with the cells inside the cornea such as keratocytes which can lead to inflammation, discomfort and discoloration. In addition, it is very hard or even impossible to remove the ink completely from the eye if required.

### SUMMARY OF THE DISCLOSURE

It is an object of the present disclosure to provide a colored ophthalmological implant for arranging within a patient's eye, a method of manufacturing a colored ophthalmological implant and an ophthalmological system for surgical treatment of a patient's eye with the colored ophthalmological implant. In particular, it is an object of the present disclosure to provide a colored ophthalmological implant for arranging within a patient's eye, a method of manufacturing a colored ophthalmological implant and an ophthalmological system for surgical treatment of a patient's eye with the colored ophthalmological implant which do not have at least some of the disadvantages of the prior art.

According to the present disclosure, these objects are addressed by the features of the independent claims. In addition, further advantageous embodiments follow from the dependent claims and the description.

According to the present disclosure, a colored ophthalmological implant for arranging within a patient's eye is specified. The ophthalmological implant comprises a base layer and eye colorant. The eye colorant is arranged to color the base layer, thereby generating the desired colored ophthalmological implant.

The base layer is made of ophthalmological tissue and provides the base for the application of the eye colorant. In other words, the base layer provides the basis or ground for the eye colorant. The eye colorant is a color or colorant and is configured to color the base layer as desired, in particular to create the desired functionality for the colored ophthalmological implant, e.g. like covering an iris defect or to form the pinhole implant.

The eye colorant is preferably made of a colorant which can be used within the human body. In other words, the eye colorant is preferably bio-compatible.

Ophthalmological tissue is tissue, which is usable within the eye of the patient. In other words, the ophthalmological tissue needs to be tissue, which is compatible with being implanted within the eye of the patient, in particular without harming the patient's eye in particular long term.

The combination of the base layer and the eye colorant form the desired colored ophthalmological implant.

According to an embodiment, the ophthalmological tissue originates from a mammalian donor tissue or from a synthetic tissue. The mammalian donor tissue is for example tissue of the patient itself, from another human, dead or alive, or from a suitable animal, dead or alive. The synthetic tissue is an artificially produced material, in particular bioengineered material, which is suitable for being used as base layer. The synthetic tissue comprises preferably collagen and / or collagen fibers. The collagen is in particular processed such that it has the desired properties for being used as base laser.

Processing collagen into shaped materials is a complex process that involves several steps, tailored to the desired final product and its application. The collagen is preferably extracted and purified. Collagen is primarily sourced from animal tissues like skin, tendons, and bones. The extraction involves breaking down the tissue and separating the collagen from other components. Methods include acid or enzyme treatments. Further, the extracted collagen is then purified to remove impurities and ensure biocompatibility. The purified collagen is preferably processed into solutions or gels, which can then be shaped using various techniques. Collagen solutions can be poured into molds to create the desired specific shapes. Collagen fibers can be spun into threads or yarns for sutures or other applications. This creates porous collagen scaffolds, often used in tissue engineering. Collagen bio-inks can be used in 3D bioprinters to create complex, customized structures. Crosslinking is a crucial step to enhance the mechanical strength and stability of the collagen material. Methods include chemical crosslinking (e.g., using glutaraldehyde) or physical crosslinking (e.g., UV irradiation). For medical applications, the collagen materials should be sterilized to eliminate any microorganisms. Methods include gamma irradiation or ethylene oxide sterilization.

The base layer is in particular at least partially transparent and the eye colorant provides the desired visual appearance, like color, reflection or absorption. Possible colors include black, white and all possible eye colors and eye patterns.

According to an embodiment comprises the base layer perforations and the eye colorant is arranged at least partially in the perforations. The base layer comprises for example a plurality of holes or cavities, which form the perforations and in which the eye colorant is at least partially arranged for coloring the base layer thereby forming the colored ophthalmological implant. The base layer is for example soaked or impregnated with the eye colorant such that the eye colorant is arranged in the perforations. The perforations are e.g. formed via a laser device, which cuts the perforations into the base layer. According to an alternative variation, the eye colorant is injected in the perforations. Further it may also be conceivable that the eye colorant is shot at high speed at the base layer, penetrates the base layer and remains in the base layer. The base layer may comprise only in a specific portion to be colored perforations. The eye colorant colors the portion to be colored of the base layer and the other portion remains uncolored as desired.

According to a further embodiment, the base layer comprises a pocket in the base layer, and wherein the eye colorant is arranged at least partially within the pocket. The pocket is a cavity within the base layer, which is configured to contain the eye colorant within the base layer. The base layer preferably comprises an access channel, through which the eye colorant is insertable and / or removable. The access channel is preferably sealed after the eye colorant has been inserted into the pocket. The pocket geometry, shape and position within the base layer is preferably selected such that the desired visual appearance of the resulting colored ophthalmological implant is realizable and / or that the portion of the base layer to be colored can be colored while the portion of the base layer not to be colored remains uncolored.

According to a further embodiment, the base layer comprises a plurality of sub-layers arranged on top of each other thereby forming the base layer. And wherein the eye colorant is at least partially arranged between at least two of the layers. The base layer comprises according to this embodiment two or more sub-layers, each being made of ophthalmological tissue, e.g. of the mammalian donor tissue and / or the synthetic tissue. Different layers may be made of different tissue. The eye colorant is arranged at least between two of the sub-layers. Different kinds of eye colorant may be arranged between different sub-layers. Thereby creating the desired visual appearance. The different sub-layers are e.g. glued or stitched, welded or fused (heat input) on top of each other such that a pocket for receiving the eye colorant remains. In another embodiment, at least one of the sub-layers is at least partially coated with eye colorant. Another sub-layer may be arranged afterward on top of the coated sub-layer, thereby forming the desired colored ophthalmological implant. Furthermore, the eye colorant is glued or printed onto at least one sub-layer of the base layer. The base layer having at least two layers with the eye colorant in between may form a sandwich structured colored ophthalmological implant. It is also conceivable that different eye colorants are arranged between different layers of the base layer, which creates together the desired visual appearance.

According to a further embodiment, the eye colorant is completely arranged within the base layer, such that the outer hull of the colored ophthalmological implant is completely formed by the base layer and / or a coating of the base layer, thereby avoiding contact of the patients eye with the eye colorant, when the colored ophthalmological implant is implanted. In other words, the eye colorant is encapsulated by the base layer, which advantageously separates the eye colorant from the eye of the patient after the implant is inserted into the eye. The likelihood that the eye colorant harmfully interacts with the eye of the patient is thereby advantageously reduced.

According to a further embodiment, the base layer comprises a coating layer, which forms at least partially, preferably entirely the outer hull of the colored ophthalmological implant. The coating is preferably at least partially made of the synthetic cornea tissue. The coating is e.g. applied after the sub layers are assembled to the base layer and / or the eye colorant is arranged or applied at or in the base layer. The coating further provides an advantageous barrier such that the eye colorant does not get in contact with the surrounding eye when the ophthalmological implant has been inserted in the patient's eye. The coating further may close the access channel to the pocket of the base layer.

According to a further embodiment, the eye colorant is arranged such that a first portion to be colored of the base layer is colored and a second portion of the base layer remains uncolored. In other words, the base layer is separated into at least two portions, namely the first portion to be colored and the second portion to remain uncolored. The eye colorant is arranged such that the first portion gets the desired color and that the second portion remains uncolored, in particular at least partially transparent. For example, the first portion is configured to be placed in the eye of the patient above the iris with respect to the line of sight or visual axis and the second portion is configured to be placed in the eye of the patient above the pupil with respect to the line of sight or visual axis. The colored ophthalmological implant thereby advantageously covers the iris portion to be covered and at the same time enables that the light can still penetrate through the implant and the pupil as desired without being absorbed by the eye colorant.

According to a further embodiment, the base layer has a central opening or wherein a central portion of the base layer is uncolored (second portion) and a portion of the base layer surrounding the central portion (first portion) is colored by the eye colorant. According to this embodiment, the ophthalmological implant forms a pinhole or ring, wherein the center portion enables that light can penetrate through the pinhole either because the base portion has a central opening or because the center portion of the base portion remains uncolored or free of eye colorant. The portion surrounding the center portion is colored with eye colorant thereby forming the pinhole or small aperture inlay.

According to a further embodiment, the geometry and / or the outer shape of the base layer is selected or determined in dependence of the individual patient's eye, in particular in dependence of the patient's individual defect to be treated by the colored ophthalmological implant. For example, the colored ophthalmological implant should cover a specific defect of the iris of the patient. The individual defect is e.g. measured in particular by using a respective measuring device, and the base portion is formed in dependence of the measurements. The measurements are processed such that the required geometry of the base layer and / or the entire implant is determined. Further, the base portion may be formed in dependence of the desired or determined pinhole geometry. The pinhole is configured to be placed in front of the pupil of the patient's eye and the required diameter of the opening of the uncolored portion is determined by the desired light screening for improving the depth of focus. The diameter of the pinhole is preferably smaller or equal to 2 mm, further, the diameter of the pinhole is more preferably smaller or equal to 1,6 mm. The diameter of the pinhole is most preferably 1,6 mm.

In general, the base layer has preferably the shape of a disc or ring, in particular wherein the outer contour and the contour of the center opening of the uncolored center portion is determined or selected in dependence of the patient individual requirements.

According to the disclosure, the maximal thickness of the colored ophthalmological implant is in a range from 5 µm to 50 µm. In a further embodiment, the maximal thickness of the colored ophthalmological implant is in a range from 5 µm to 30 µm, preferably in a range from 5 µm to 20 µm. The maximal thickness is the largest extension between two opposing points, each point being arranged at the outmost outer surfaces of the ophthalmological implant. For example, in case the ophthalmological implant has a disc shape it comprises at least three outer surface portions forming together the outer hull. The three surfaces are the two opposing relatively large surfaces and the connecting relatively small lateral surface. The maximal thickness is the largest extension between the two opposing points, one being arranged at the first large surface and the other point being arranged at the second large surface. It is in particular advantageous that the colored ophthalmological implant is as required thin, thereby avoiding a refractive effect of the ophthalmological implant.

According to a further embodiment, the eye colorant comprises composite material particles, which comprise coloring pigments of at least one color and, a matrix material, which at least partially embeds the coloring pigments, thereby forming the composite material particles. The composite material particles comprise according to the present disclosure the coloring pigments and the matrix material. The coloring pigments, which define at least partially the color of the eye colorant, are embedded in the matrix material, which is preferably a solid material. The matrix material, which is for example, at least partially transparent, surrounds at least partially, preferably entirely, the coloring pigments. In case the matrix material is fully transparent, the color of the eye colorant is only determined by the coloring pigments. The combination of the coloring pigments and the matrix material in the composite material particles enable to create advantageously realistic and imitation of eye color using such eye colorant. Further, the eye colorant according to the present disclosure is advantageously stable and long lasting. Different composite material particles each comprising different coloring pigments may be combined to create the desired color and structure of the eye colorant for the eye of the patient. The eye colorant as specified is, for example, injected into the cornea of a patient at a predefined portion of the cornea of the eye. The portion is, for example, ring shaped or partially ring shaped, has the shape of a tunnel and covers for example, the desired defected portion of the iris of the eye of the patient.

In an embodiment, the eye colorant further comprises a carrier liquid, in which the composite material particles are distributed. The composite material particles are arranged and distributed in the carrier liquid, which simplifies the handling of the eye colorant. In an embodiment, the carrier liquid comprises a balanced salt solution, a buffered saline solution and / or sterile intraocular irrigating solution, preferably balanced salt solution enriched with bicarbonate, dextrose, and/or glutathione. In an embodiment, the carrier liquid may be BSS^{®} or BSS^{®} PLUS from Alcon Inc. In an embodiment, the carrier liquid is at least partially a sodium hyaluronate solution. The carrier liquid may further comprise at least one of: Polyglutamic Acid, glycerin, sorbitol, propylene glycol, or butylene glycol.

In an embodiment, the eye colorant further comprising coloring pigments, which are distributed in the carrier liquid and / or additional particles, which are distributed in the carrier liquid. According to this embodiment, coloring pigments of, for example, at least one color are additionally distributed in the carrier liquid. Further, additional particles of, for example, at least one kind, are distributed in the carrier liquid.

In an embodiment, at least some of the additional particles have a reflective surface and / or wherein at least some of the additional particles have an absorbing property. A reflective surface is a surface, which at least partially, preferably entirely, reflects incident light. Such a reflective / shiny surface advantageously helps to recreate the desired eye color and / or eye color distribution (natural iris appearance) by the eye colorant comprising such additional particles. Further, an absorbing particle is a particle, which at least partially, preferably entirely, absorbs incident light. In an embodiment, the additional particles, comprising the reflective surface, are made of titanium dioxide. Titanium dioxide has a refractive index that is significantly higher than that of most organic substances used to bind colors. This means that the additional particles comprising titanium dioxide scatter light effectively, resulting in a well-covered white color. Here, the optimum size of the pigments is in the range of 200 nm to 300 nm. In a further embodiment, the additional particles comprising the reflective surface are made of aluminum oxide and / or silicon oxide. Absorbing particles comprise e.g. carbon-based pigments.

In a further embodiment, the carrier liquid is made of biocompatible material and / or a transparent material and / or medically active material. According to this embodiment, the carrier liquid may additionally or alternatively provide the advantages as described above with respect to the biocompatibility, transparency and / or medical or pharmaceutical activity.

In an embodiment, the matrix material is made of a thermoplastic material, preferably made of a polymethyl methacrylate material, or wherein the matrix material is made of a synthetic polymer material, preferably of a polyorganosiloxane material. A combination of the thermoplastic material and the synthetic polymer material is also conceivable.

In an embodiment, the composite material particles have an average equivalent diameter of below 25 micrometers, preferably below 10 micrometers, more preferably below 5 micrometers. In an embodiment, the composite material particles have an average equivalent diameter of above 1 micrometer, preferably above 2 micrometers, more preferably above 4 micrometers. In case the composite material particles are too small, they might move within the cornea towards undesired outer or inner portions of the cornea, this further depends on other properties of the particles. In case the composite material particles are too large, they might be perceived as a disturbing element in the cornea of the eye. Further, large particles may cause unwanted negative reactions. The composite material particles having the average equivalent diameter as described above provide the desired properties. The equivalent diameter may be the equivalent spherical diameter. The average equivalent diameter is the average diameter of a number of particles of a sample of the composite material particles. In an embodiment, a mesh size of 1000 or finer is used for filtering and / or determining of the desired composite material particle size.

In an embodiment, the composition of the coloring pigments is configured to be selected in dependence of a current iris structure and / or a current iris color distribution of the eye of the patient. In a further embodiment, the composition of the coloring pigments is configured to be selected in dependence of a target iris structure and / or a target iris color distribution selected by the patient.

In a further embodiment, in case, when the base layer is made of mammalian donor tissue, it is in particular made of cornea tissue of a donor eye, cornea tissue of the patient's eye, stroma tissue of a donor eye, stroma tissue of the patient's eye, bowman's layer of a donor eye. Other suitable mammalian membranes, like the Descemet membrane or membranes not belonging to the human eye are also conceivable. These kinds of tissue provide advantageously the required properties for the base layer.

Further possible synthetic tissues may include synthetic peptides and polypeptides, which are designed to mimic the amino acid sequences and triple-helix structure of collagen. Further possible synthetic tissue may include polymeric materials, like Poly(lacticco-glycolic acid) (PLGA), Polycaprolactone (PCL), Polyethylene glycol (PEG), Silk Fibroin, and / or Hyaluronic Acid (HA).

According to a further aspect of the present disclosure a method of manufacturing a colored ophthalmological implant as described above and hereinafter is specified. The method comprises at least the following steps.

Providing, an ophthalmological tissue, wherein the ophthalmological tissue originates is in particular from a mammalian donor tissue or from a synthetic cornea tissue.

Processing the base layer from the provided ophthalmological tissue. In other words, the provided ophthalmological tissue is processed or machined into the base layer. For example, the ophthalmological tissue is cut into the desired shape to form the base layer.

Coloring the base layer with eye colorant, thereby forming the colored ophthalmological implant. In this step, the eye colorant is applied or arranged at or within the base layer to color the base layer.

According to a further embodiment, the method may include the additional step of coating the colored base layer, with a coating layer, thereby forming a coated colored ophthalmological implant. The coating layer is in particular made at least partially of a synthetic tissue, in particular as described above.

According to a further embodiment, the step of processing the base layer comprises cutting the outer contour of the base layer out of the ophthalmological tissue and/or forming the base layer such that the eye colorant is applicable as desired. For example, the pocket or the perforations for the eye colorant are formed.

According to a further embodiment, the method comprises the step of measuring the patient's eye and wherein the step of processing the base layer is performed by using the determined measurements of the patient's eye. The measurements may include the position and orientation of an iris and / or eye defect or condition.

According to a further embodiment, the step of coloring the base layer comprises inserting the eye colorant in perforations and / or in at least one pocket of the base layer, coating at least partially the base layer or coating at least one sub-layer, which forms partially the base layer with eye colorant and / or impregnate at least partially the base layer with eye colorant.

According to a further aspect of the present disclosure an ophthalmological system for surgical treatment of a patient's eye with the colored ophthalmological implant is specified. The ophthalmological system comprises an ophthalmological device and an implantation device.

The ophthalmological device comprises a laser source configured to generate a pulsed laser beam, a focusing optics configured to make the pulsed laser beam converge onto a focal spot in the patient's eye, a scanner system configured to deflect the pulsed laser beam to target locations in the patient's eye, and an electronic circuit configured to control the scanner system for cutting an implant pocket inside the patient's eye, in which the colored ophthalmological implant as described above and hereinafter is to be implanted.

The implantation device is configured to implant the colored ophthalmological implant in the implant pocket inside the patient's eye.

### BRIEF DESCRIPTION OF THE DRAWINGS

The herein described disclosure will be more fully understood from the detailed description given herein below and the accompanying drawings, which should not be considered limiting to the invention described in the appended claims. The drawings in which:
- **Figure 1**: shows a colored ophthalmological implant according to a first embodiment
- **Figure 2**: shows a colored ophthalmological implant according to a second embodiment;
- **Figure 3**: shows a plurality of colored ophthalmological implants according to further embodiments;
- **Figure 4**: shows a colored ophthalmological implant according to a further embodiment;
- **Figure 5**: shows a colored ophthalmological implant according to a further embodiment;
- **Figure 6**: shows an eye colorant according to a first embodiment;
- **Figure 7**: shows an eye colorant according to a first embodiment;
- **Figure 8**: shows an eye of a patient according to a first embodiment prior to treatment;
- **Figure 9**: shows the eye of the patient according to the first embodiment after treatment;
- **Figure 10**: shows a schematic view of an ophthalmological system;
- **Figure 11**: shows a block diagram illustrating schematically different steps of a method for manufacturing a colored ophthalmological implant according to a first embodiment;
- **Figure 12**: shows a section view of an eye of a patient during treatment with the ophthalmological system, in particular of forming the pocket for inserting the colored ophthalmological implant;
- **Figure 13**: shows a perspective view of an eye of a patient during treatment with the ophthalmological system, in particular of inserting in the pocket the colored ophthalmological implant;
- **Figure 14**: shows a perspective view of an eye of a patient after treatment with the ophthalmological system, in particular with the colored ophthalmological implant in the pocket of the cornea;
- **Figure 15**: shows a front view of an eye of a patient after treatment with the ophthalmological system, in particular with the colored ophthalmological implant in the pocket of the cornea;
- **Figure 16**: shows schematically the extraction of a membrane out of a donor eye.

### DESCRIPTION OF THE EMBODIMENTS

Reference will now be made in detail to certain embodiments, examples of which are illustrated in the accompanying drawings, in which some, but not all features are shown. Indeed, embodiments disclosed herein may be embodied in many different forms and should not be construed as limited to the embodiments set forth herein; rather, these embodiments are provided so that this disclosure will satisfy applicable legal requirements. Whenever possible, like reference numbers will be used to refer to like components or parts.

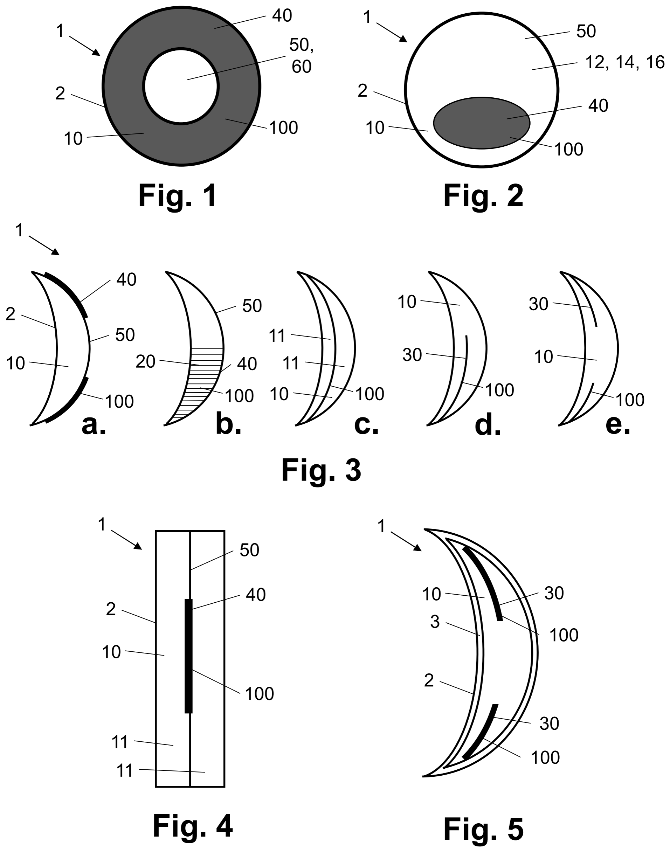

The **Figures 1** to **Figures 5** show a plurality of different embodiments of colored ophthalmological implants 1, for inserting in a cornea 220 of an eye 210 of a patient.

**Figure 1** shows the colored ophthalmological implant 1 with its outer hull or contour 2. The colored ophthalmological implant 1 has a round disclike shape and comprises a base layer 10 and respective eye colorant 100, which colors the first portion 40 of the base layer 10, in particular the radial outer portion. The colored ophthalmological implant 1 further comprises a central opening 60. In another embodiment, the central portion or second portion 50 of such a colored ophthalmological implant 1 may remain uncolored.

**Figure 2** shows the colored ophthalmological implant 1 having a different shape, the outer hull or contour 2 still has a round disklike shape but the first portion 40 to be colored is arranged not coaxially with the outer contour and the second uncolored portion 50 of the colored ophthalmological implant 1. The eye colorant 100 is only arranged at the first portion 40. Furthermore, the first portion 40 to be colored has an elliptical shape when viewed from above, other shapes are of course also conceivable. This colored ophthalmological implant 1 may be used to cover an iris defect 252 of a patient's eye 210. At the same time, light may further penetrate through the second uncolored portion 50 such that it can enter the pupil of the patient's eye 210. Figure 2 further indicates that the base layer 10 is made of ophthalmological tissue 12, in particular mammalian donor tissue 14 or synthetic tissue 16.

**Figure 3** shows a plurality of different embodiments of the colored ophthalmological implant 1. According to the embodiment of **Figure 3a****,** the eye colorant 100 is applied e.g. glued or coated on the outer surface of the base layer 10. It is further visible that the first outer portion 40 is colored and that the central portion 50 remains uncolored. **Figure 3b** shows that the base layer 10 comprises a plurality of perforations 20 at the first portion 40 to be colored. The second uncolored portion 50 is perforation free. The eye colorant 100 is arranged in the perforations 20 to color the base layer 10 as desired. **Figure 3c** shows that the base layer 10 comprises a plurality of sub-layers 11 which are arranged on top of each other and which form the base layer 10. The eye colorant 100 may be arranged between at least two of the sub-layers 11. **Figures 3d and 3e** shows a pocket 30 arranged in the base layer 10. The pocket 30 according to Figure 3e is arranged symmetrical and the pocket 30 according to Figure 3d is arranged asymmetrical. The eye colorant 100 may be arranged in the pocket 100.

**Figure 4** shows a further embodiment of the colored ophthalmological implant 1. The base layer 10 is according to this embodiment made of a plurality of sub-layers 11 and the eye colorant 100 is arranged between two sub-layers 11. Further it is visible that the first colored portion 40 does not extend entirely across the implant 1 the outer second uncolored portion 50 surrounds the first colored portion 40. The base layer 10 forms the outer hull 2 of the colored ophthalmological implant 1. Further, the shape of implant 1 differs from the other embodiments, in particular in that the cross section has not a lens shape but has a rectangular shape. This highlights that different shapes and in particular also different cross sections are conceivable.

**Figure 5** shows a further embodiment of the colored ophthalmological implant 1. The colored ophthalmological implant 1 comprises a pocket 30 surrounding the central portion. The eye colorant 100 is arranged in the pocket 30 thereby forming a pinhole implant. The base layer 10 is further coated with a coating layer 3 such that only the coating layer 3 gets in contact with the cornea 220 of the patient's eye 210 after being implanted in the patient's eye.

Further, it is to be noted that the coating via the coating layer 3 as shown in Figure 5 may be applied at any different embodiment of the implant 1, e.g. as shown in the Figures 1 to 4.

The implants 1 are for example shaped as lenticules. A further preferred variant is flat or ribbon-shaped (e.g. a material ribbon that the surgeon can cut to size and insert into the pocket in the patients eye). Furthermore, refractive implants with a lenticular shape create at the same time a myopic advantage for the respective eye, which further optimizes the depth of focus.

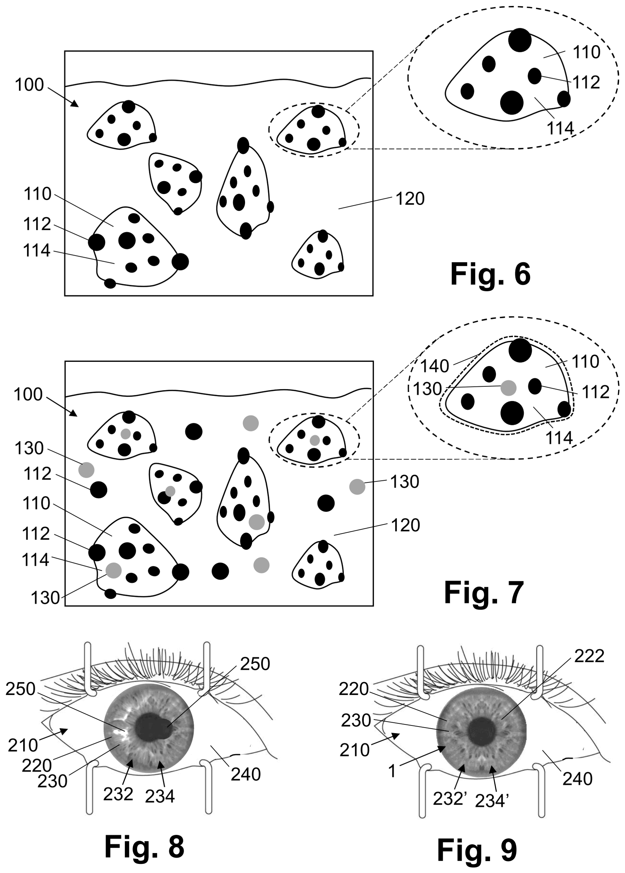

**Figure 6** shows an eye colorant 100 according to a first embodiment. The eye colorant 100 comprises a plurality of composite material particles 110, which are distributed in a carrier liquid 120. The composite material particles 110 comprise matrix material 114 and coloring pigments 112. The coloring pigments 112 are at least partially embedded in the matrix material 114, thereby forming the composite material particles 110. The composite material particles 110 have all kinds of different shapes, which are not limited to any specific shape. **Figure 6** shows the eye colorant 100 schematically in a container. The eye colorant 100 as shown in **Figure 6** is not at scale, it is illustrated at a larger scale. **Figure 6** further shows a detailed view of a single composite material particle 110. In particular, the detailed view shows that the arbitrary arrangement of the coloring pigments 112 within the matrix material 110. The coloring pigments 112 determine at least partially the color of the eye colorant 100; in case the matrix material 114 and the carrier liquid 120 is fully transparent, the color of the eye colorant 100 is entirely determined by the coloring pigments 112. The coloring pigments 112 are for example merged or applied on the matrix material 114 in a liquid phase of the matrix material 114.

For example, the matrix material 114 is liquefied, e.g. melted and the coloring pigments 112 are added to the matrix material 114. In a next step, the matrix material 114 comprising the coloring pigments 112 is solidified, e.g. cured and the particles 110 are formed, for example by breaking the cured matrix material 114 into the desired size of the particles 110.

In another embodiment, the liquid matrix material 110 comprising the coloring pigments 112 is transformed into droplets having the desired size and cured afterwards for forming the composite material particles 110. The composite material particles 110 may be post-processed at a later stage, for example polished or coated.

The composite material particles 110 have an average equivalent diameter of below 25 micrometers, preferably of below 10 micrometers, more preferably of below 5 micrometers. Further, the composite material particles 110 have an average equivalent diameter of above 1 micrometer, preferably above 2 micrometers, more preferably above 4 micrometers. The average equivalent diameter is the average diameter of a number of particles of a sample of the composite material particles 110. The average equivalent diameter is for example the volume-equivalent sphere diameter or the surface-equivalent sphere diameter. As shown in **Figure 6****,** the coloring pigments 112 are of a single color within one single composite material particles 110 and within the plurality of the shown composite material particles 110. In another embodiment, one single composite material particles 110 may comprise a plurality of coloring pigments 112 of different colors. In a further embodiment, the eye colorant 100 comprises different composite material particles 110 with different coloring pigments 112.

**Figure 7** shows a second embodiment of an eye colorant 100. This embodiment deviates from the first embodiment shown in **Figure 6** in that additional coloring pigments 112 are distributed in the carrier liquid 120 and, in that additional particles 130 are distributed in the carrier liquid 120. The coloring pigments 112 in the carrier liquid 120 additionally increase the possible variety of colors of the eye colorant 100. The additional particles 130 may comprise a light reflective or a light absorbing surface. The additional particles are for example carbon based and / or comprise titan oxide and / or iron oxide. The additional particles 130 in the carrier liquid 120 and / or in the composite material particles 110 additionally increase the possible variety of colors of the eye colorant 100, in particular with respect to brightness, shininess or darkness.

The detailed view of **Figure 7****,** showing one single composite material particle 110, additionally shows a colorant coating 140 around the single composite material particle 110. The colorant coating 140, which, according to this embodiment entirely surrounds the composite material particles 110, is made of, for example, a biocompatible material or a medical or pharmaceutical active material. In a further embodiment, the additional particles 130 and / or the coloring pigments 112 distributed in the carrier liquid 120 may also be coated, in particular with the bio-compatible and / or medical or pharmaceutical active coating.

Other kinds of eye colorants 100 for coloring the base layer 10 are of course also conceivable.

**Figure 8** shows an eye 210 of a patient 200 according to a first embodiment prior to treatment with the colored ophthalmological implant 1. The eye 210 shows the sclera 240, the transparent cornea 220 and the natural iris 230 with its current iris structure and its current iris color distribution. The iris 230 as shown in **Figure 8** comprises white spots 250, which affects the visual appearance of the iris 230. Other or additional spots on the iris 230 like a remaining after melanoma removal are also conceivable. **Figure 8** further shows that the iris 230 comprises a defect 252, such that the iris 230 does not have a perfect ring shape. The iris defect 252 may result from a congenital disease such as coloboma. Such diseases or defects of the iris 230 or the cornea 220 of the eye affect the visual appearance and the functionality of the eye 210 of the patient 200. The colored ophthalmological implant 1 may be used to amend this visual appearance, as shown e.g. in **Figure 9****.**

**Figure 9** shows the eye 210 of the patient 200 according to the first embodiment after treatment with the colored ophthalmological implant 1. The colored ophthalmological implant 1 has been inserted in a pocket in the cornea 220 in front of the iris 230, with respect to the visual axis of the eye 210. The colored ophthalmological implant 1 recreates the visual appearance of the desired color and structure of the iris 230 of the eye 210. The white spot 250 and the iris defect 252 are not removed, but they are covered by the colored ophthalmological implant 1. The colored ophthalmological implant 1 is according to this embodiment further used to recreate a target iris structure and a target iris color distribution. In this embodiment, the target iris structure and the target iris color distribution corresponds to the current iris structure and the current iris color distribution without the white spot 250 and without the iris defect 252. The colored ophthalmological implant 1 advantageously creates the desired visual appearance of the eye 210 of the patient 200 and may further treat any defects of the eye 210 of the patient 200.

The colored ophthalmological implant 1 may further be used as a pinhole diaphragm to increase the depth of focus, in particular to treat insufficiencies with the depth of field.

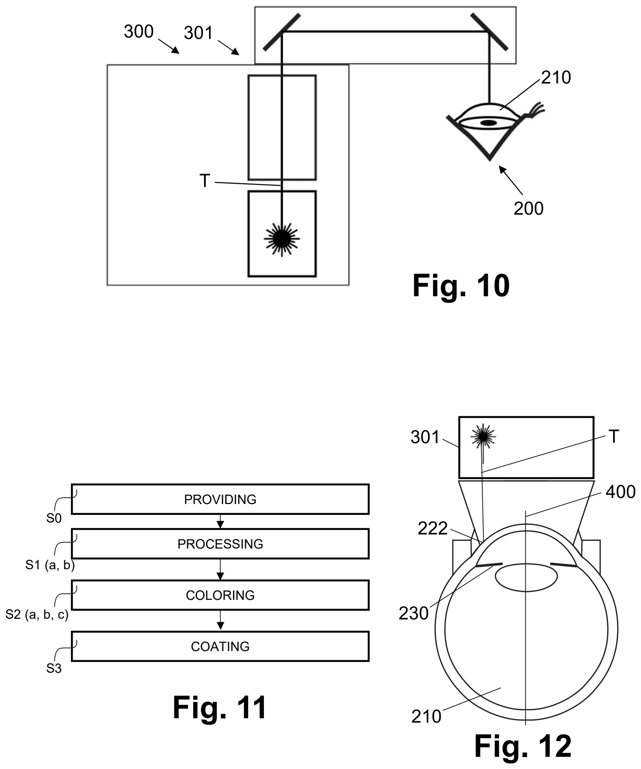

**Figure 10** schematically illustrates an ophthalmological system 300 and a patient 200, in particular an eye 210 of the patient 200, which is treated with the ophthalmological system 300, in particular with an ophthalmological device 301. The ophthalmological device 301 comprises a base station. The base station is for example a fixed or mobile apparatus. The ophthalmological device 301 has a treatment laser source arranged in the base station, which generates a treatment laser beam T. The base station further includes, for example, a power supply and other auxiliary subsystems necessary for operation of the ophthalmological device 301. The treatment laser source is configured, for example, to generate an infrared treatment laser beam T having a wavelength 780 nm to 1100 nm. In an embodiment, the treatment laser beam T is a pulsed laser beam. In an embodiment, the treatment laser source is configured to generate femtosecond laser pulses, which have pulse widths of typically from 10 fs to 1000 fs (1 fs = 10^-15 s). The ophthalmological device 301comprises a laser applicator or application head. The laser applicator is designed to guide the treatment laser beam T into or onto an eye 210 of a patient 200. The laser applicator can comprise focusing optics configured to focus the treatment laser beam T onto one or more treatment points inside or on the eye 210, in particular the cornea for a pointwise tissue disruption or ablation. The ophthalmological device 301 may comprise an ophthalmological patient interface. The laser applicator is preferably fixed onto the eye 210 by means of the ophthalmological patient interface, which is coupled to the eye for example using negative pressure.

The ophthalmological device 301 optionally includes a user interface comprising, for example, one or more user input devices, such as a keyboard, and one or more output devices, such as a display (not shown in Figures 10). The display may also be an input device. The user interface is configured to receive user inputs from an eye treatment professional, in particular based on, or in response to, information displayed to the eye treatment professional using the one or more output devices.

**Figure 11** shows a block diagram illustrating schematically different steps of a method for manufacturing the colored ophthalmological implant 1. The colored ophthalmological implant 1 may be inserted or implanted in a portion of the cornea 220 of the eye 210 of the patient 200. The block diagram comprises the steps S0 to S3, additional or alternative steps are also conceivable.

In step S0, the ophthalmological tissue 12, in particular the mammalian donor tissue 14 or the synthetic tissue 16 is provided. Different sub-layers 11 may be made of different tissue. The mammalian donor tissue 14, it is in particular made of cornea tissue of a donor eye, cornea tissue of the patient's eye, stroma tissue of a donor eye, stroma tissue of the patient's eye, bowman's layer of a donor eye, bowman's layer of the patients' eye. Other suitable mammalian membranes, like the Descemet membrane are also conceivable. These kinds of tissue provide advantageously the required properties for the base layer.

In step S1, the provided ophthalmological tissue 12 is processed into the base layer 10. For example, the provided ophthalmological tissue 12 is cut and / or formed (step S1a) into the desired patient individual shape, e.g. into a disklike shape having a specific outer contour to cover the patient individual iris defect 252. In a further sub step S1b, the base layer 10 is machined such that the eye colorant 100 is applicable as desired. For example, the perforations 20 and / or the pocket 30 are formed into the base layer 10.

In step S2, the base layer 10 is colored with the eye colorant 100 thereby forming the colored ophthalmological implant 1. The step S2 may comprise a plurality of different or alternative sub-steps. For example, in step S2a, the eye colorant 100 is inserted into the perforations 20 of the base layer 10 for coloring it. In step S2b, the base layer 10 is at least partially coated with eye colorant 100. In step S2c, the base layer 10 is at least partially impregnated with eye colorant 100. Other coloring techniques are of course also conceivable.

In optional step S3, the colored base layer 10 is coated with a coating layer 3, which creates a coated colored ophthalmological implant 1 as shown e.g. in Figure 5.

It should be noted that, in the description, the sequence of the steps has been presented in a specific order, one skilled in the art will understand, however, that the order of at least some of the steps could be altered or some steps could be skipped, without deviating from the scope of the disclosure.

**Figure 12** shows a longitudinal section view of the eye 210 of a patient 200, which extends along its central axis 400. **Figure 12** further shows the different components of the eye 210. The eye 210 comprises the cornea 220, the iris 230 and the sclera 240. According to this embodiment, the ophthalmological device 301, as for example described with reference to **Figure 10****,** is applied on the eye 210, which uses the treatment laser beam T to cut an implant pocket 222 into the cornea 220 of the eye 210. Alternatively, or additionally a scalpel or similar tissue separating instruments may be used to cut the implant pocket 222.

The implant pocket 222 may cover only a portion of the cornea 220, which is for example affected by a disease or iris defect 252 or may have a circular or shape for covering the entire iris 230.

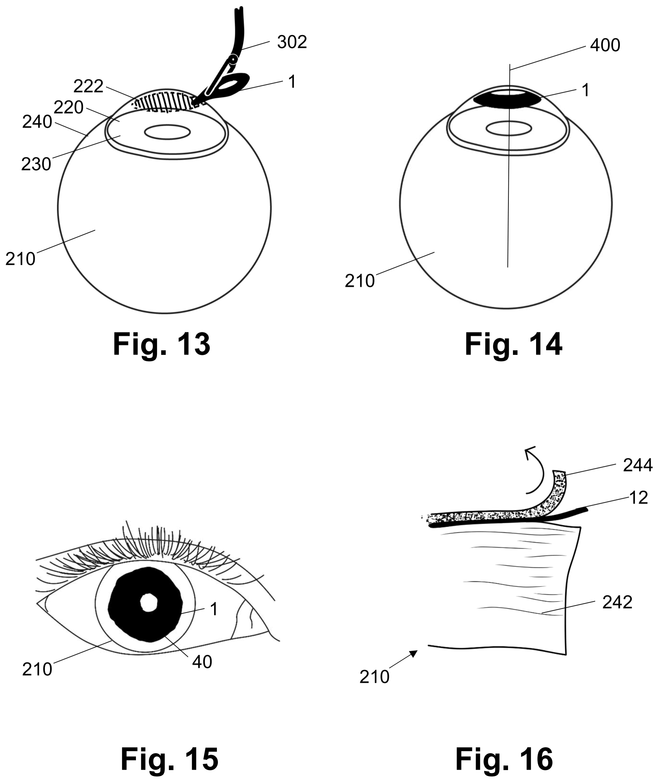

**Figure 13** shows a perspective view of the eye 210 of the patient 200 during the inserting step of the colored ophthalmological implant 1 into the implant pocket 222. The implant pocket 222, which has been previously cut as described above with respect to Figure 12, is filled with the colored ophthalmological implant 1 by using the implantation device 302. The implantation device 302 and the ophthalmological device 301 form the ophthalmological system 300. The implantation device 302 is e.g. an ophthalmological forceps, which pushes the colored ophthalmological implant 1 through an access channel into the implant pocket 222. Other devices may also be conceivable as implantation devices 302. Other implantation techniques are of course also conceivable and may be used for inserting the implant 1 at the desired spot.

**Figure 14** shows a perspective view of the eye 210 of the patient 200 after the implantation of the colored ophthalmological implant 1 into the implant pocket 222. According to this embodiment, the colored ophthalmological implant 1 serves as a pinhole diaphragm for increasing the depth of focus for treating insufficiencies with the depth of field. It is advantageously visible that the colored ophthalmological implant 1 is arranged coaxially with the central axis 400 of the eye 210 above the pupil. The central portion of the colored ophthalmological implant 1 is uncovered such that light can still penetrate through.

**Figure 15** shows a front view of the eye 210 of the patient 200 after the implantation of the colored ophthalmological implant 1 into the implant pocket 222. Figure 15 further shows that the radial outer contour of the portion to be colored is irregular for a more realistic blending with the natural color of the iris.

**Figure 16** shows a section view of an eye 210. Figure 16 shows the stroma tissue 242, the epithelium tissue 244 of the eye 210. The epithelium 244 is removed for accessing the bowman membrane, which is e.g. removed and used as ophthalmological tissue 12 for producing the base layer 10.

### LIST OF REFERENCE SYMBOLS

- 1: colored ophthalmological implant
- 2: outer hull
- 3: coating layer
- 10: base layer
- 11: sub-layer
- 12: ophthalmological tissue
- 14: mammalian donor tissue
- 16: synthetic tissue
- 20: perforation
- 30: pocket
- 40: first portion to be colored
- 50: second portion uncolored
- 60: central opening
- 100: eye colorant
- 110: composite material particles
- 112: coloring pigments
- 114: matrix material
- 120: carrier liquid
- 130: additional particles
- 140: colorant coating
- 200: patient
- 210: eye
- 220: cornea
- 222: implant pocket
- 230: iris
- 240: sclera
- 242: stroma
- 244: epithelium
- 250: white spot
- 252: iris defect
- 300: ophthalmological system
- 301: ophthalmological device
- 302: implantation device
- 310: patient interface
- 400: central axis

- T: treatment laser beam
- S0: Providing
- S1: Processing
- S1a: Cutting
- S1b: Machining
- S2: Coloring
- S2a: Inserting
- S2b: Coating
- S2c: Impregnating
- S3: Coating

## Claims

1. Colored ophthalmological implant (1) for arranging within a patient's eye (210), the colored ophthalmological implant (1) comprising:
a. a base layer (10), which is made of ophthalmological tissue (12); and
b. eye colorant (100), which is configured to color the ophthalmological implant (1); and
wherein the eye colorant (100) is arranged to color the base layer (10), thereby generating the desired colored ophthalmological implant (1) and
wherein the maximal thickness of the colored ophthalmological implant (1) is in a range from 5 µm to 50 µm.

2. The colored ophthalmological implant (1) according to claim 1, **wherein** the ophthalmological tissue (12) originates from a mammalian donor tissue (14) or from a synthetic tissue (16.

3. The colored ophthalmological implant (1) according to any one of the preceding claims, **wherein** the base layer (10) comprises perforations (20) and the eye colorant (100) is arranged at least partially within the perforations (20).

4. The colored ophthalmological implant (1) according to any one of the preceding claims, **wherein** the base layer (10) comprises a pocket (30) in the base layer (10), and wherein the eye colorant (100) is arranged at least partially within the pocket (30).

5. The colored ophthalmological implant (1) according to any one of the preceding claims, **wherein** the base layer (10) comprises a plurality of sub-layers (11) arranged on top of each other, and wherein the eye colorant (100) is at least partially arranged between at least two of the sub-layers (11).

6. The colored ophthalmological implant (1) according to any one of the preceding claims, wherein the eye colorant (100) is completely arranged within the base layer (10), such that the outer hull (2) of the colored ophthalmological implant (1) is completely formed by the base layer (10) and / or a coating (3), thereby avoiding contact of the patients eye (210) by eye colorant (100), when the colored ophthalmological implant (1) is implanted in the patients eye (210).

7. The colored ophthalmological implant (1) according to any one of the preceding claims, **wherein** the eye colorant (100) is arranged such that a first portion (40) to be colored of the base layer (10) is colored and a second portion (50) of the base layer (10) remains uncolored.

8. The colored ophthalmological implant (1) according to any one of the preceding claims, **wherein** the base layer (10) has a central opening (60) or wherein a central portion of the base layer (10) is uncolored and a portion of the base layer (10) surrounding the central portion is colored by the eye colorant (100).

9. The colored ophthalmological implant (1) according to any one of the preceding claims, **wherein** the maximal thickness of the colored ophthalmological implant (1) is in a range from 5 µm to 30 µm, preferably in a range from 5 µm to 20 µm.

10. The colored ophthalmological implant (1) according to any one of the preceding claims, **wherein** the eye colorant (100) comprises:
a. composite material particles (110) comprising:
i. coloring pigments (112) of at least one color and,
ii. a matrix material (114), which at least partially embeds the coloring pigments (112), thereby forming the composite material particles (110).

11. The colored ophthalmological implant (1) according to any one of the preceding claims, **wherein** the base layer (10) is made of at least one of: cornea tissue of a donor eye, cornea tissue of the patients eye (210), stroma tissue of a donor eye, stroma tissue of the patients eye (210), bowman's layer of a donor eye, bowman's layer of the patients eye (210).

12. A method of manufacturing a colored ophthalmological implant (1) according to any one of the preceding claims, wherein the method comprises the steps of:
a. Providing (S0), an ophthalmological tissue (12);
b. Processing (S1) the base layer (10) out of the provided (S1) ophthalmological tissue (12);
c. Coloring (S2) the base layer (10) with eye colorant (100), thereby forming the colored ophthalmological implant (1),
wherein the maximal thickness of the colored ophthalmological implant (1) is in a range from 5 µm to 50 µm.

13. The method of manufacturing the colored ophthalmological implant (1) according to claim 12, further comprising the step of:
a. Coating (S3) the colored base layer (10), with a coating layer (3), thereby forming a coated colored ophthalmological implant (1).

14. The method of manufacturing the colored ophthalmological implant (1) according to claim 12 or claim 13, **wherein** the step of processing (S1) the base layer (10) comprises at least one of:
a. Cutting (S1a) the outer contour of the base layer (10) out of the ophthalmological tissue (12); or
b. Machining (S1b) the base layer (10) such that the eye colorant (100) is applicable.

15. The method of manufacturing the colored ophthalmological implant (1) according to any one of the claims 11 to 14, **wherein** the step of coloring (S2) the base layer (10) comprises at least one of:
a. Inserting (S2a) the eye colorant (100) into perforations (20) and / or in at least one pocket (30) of the base layer (10);
b. Coating (S2b) at least partially the base layer (10) or coating at least one sub-layer (11), which forms partially the base layer (10) with eye colorant (100); or
c. Impregnating (S2c) at least partially the base layer (10) with eye colorant (100).

16. An ophthalmological system (300) for surgical treatment of a patient's eye (210) with the colored ophthalmological implant (1), the ophthalmological system (300) comprising:
a. an ophthalmological device (301) comprising:
i. a laser source configured to generate a pulsed laser beam (T);
ii. a focusing optics configured to make the pulsed laser beam (T) converge onto a focal spot (S) in the patient's eye (210);
iii. a scanner system configured to deflect the pulsed laser beam (T) to target locations in the patient's eye (210); and
iv. an electronic circuit configured to control the scanner system for cutting an implant pocket (222) inside the patient's eye (210), in which the colored ophthalmological implant (1) according to any one of the preceding claims 1 to 11 is to be implanted; and
b. an implantation device (302), which is configured to implant the colored ophthalmological implant (1) in the implant pocket (222) inside the patient's eye (210).
